# EUROPEAN PATENT APPLICATION

(11) **EP 2 584 361 A1**
(43) Date of publication of application: **24.04.2013**
(21) Application number: 11795675.5
(22) Date of filing: 13.06.2011
(51) Int. Cl.: G01N 33/576, G01N 33/573

(54) **MARKER FOR DETECTION AND/OR DISCRIMINATION OF NON-ALCOHOLIC STEATOHEPATITIS, METHOD FOR DETECTION AND/OR DISCRIMINATION OF NON-ALCOHOLIC STEATOHEPATITIS, AND KIT FOR USE IN THE METHOD**

(30) Priority: 17.06.2010 JP 2010137795
(71) Applicant: National University Corporation Chiba University, Chiba-shi Chiba 263-8522 (JP); Nitto Boseki Co., Ltd, Fukushima-shi, Fukushima 960-8161 (JP); Nittobo Medical Co., Ltd., Tokyo 102-0083 (JP)
(72) Inventor: NOMURA Fumio, Chiba-shi Chiba 260-8670 (JP); NISHIMURA Motoi, Chiba-shi Chiba 260-8670 (JP); KATAYAMA Katsuhiro, Tokyo 102-0073 (JP); KIYOKAWA Iwao, Koriyama-shi Fukushima 963-8061 (JP)
(74) Representative: HOFFMANN EITLE
(86) International application number: PCT/JP2011/063446
(87) International publication number: WO 2011/158769

(57) **Abstract**

The detection of a non-alcoholic steatohepatitis patient and the accurate discrimination between a normal person and a non-alcoholic steatohepatitis patient can be achieved by measuring alanine-glyoxylate amino transferase in a sample.

## Description

### Technical Field

The present invention relates to a marker for detection and/or differentiation of non-alcoholic steatohepatitis, a method for detecting and/or differentiating non-alcoholic steatohepatitis, and a kit for use in the method. Specifically, the present invention relates to a marker for detection and/or differentiation of non-alcoholic steatohepatitis that is suitable for the detection of non-alcoholic steatohepatitis, which has previously been difficult to diagnose by an approach other than liver biopsy, and for the differential diagnosis thereof from fatty liver, a method for detecting and/or differentiating non-alcoholic steatohepatitis, and a kit for use in the method.

### Background Art

With recent changes in eating habits, soaring numbers of patients have suffered from steatohepatitis, which are found to have fatty liver accompanied by inflammation similarly to fatty liver or alcoholic hepatitis in the histopathological examination of livers, even though they do not drink alcohols. Such a new category of disease is collectively called non-alcoholic fatty liver disease (NAFLD). The non-alcoholic fatty liver disease consists of non-alcoholic fatty liver (simple steatosis) and non-alcoholic steatohepatitis (hereinafter, also referred to as NASH), which is accompanied by hepatic fibrosis and progresses into cirrhosis or cancer of the liver. NASH is a relatively new disease concept proposed by Ludwig et al. in 1980 (Non Patent Literature 1).
Heretofore, simple steatosis has been regarded as a disease with favorable prognosis. By contrast, non-alcoholic steatohepatitis (NASH) has reportedly been found analytically with a frequency of 6.3% in the postmortem dissection of 351 donor bodies having no history of alcohol intake, showing the presence of NASH in NAFLD (Non Patent Literature 2).
NASH is the most general chronic liver disease in the USA. Also in Japan, this disease, which exhibits an advanced stage of fat accumulation in the liver or liver damage despite no history of alcohol intake or negativity for hepatitis virus, has been addressed since the 1990s with increases in the number of patients with obesity or diabetes mellitus. This disease has been regarded in recent years as a liver disease that progresses evidently independently of other lifestyle-related diseases such as diabetes mellitus, though the disease is often seen in obese persons and also strongly related to insulin resistance.

NASH is a progressive disease leading to liver cirrhosis from hepatitis and requires medical intervention at an earlier stage than the development of liver cirrhosis, which is poorly reversible. By contrast, simple steatosis, which constitutes a majority of NAFLDs, is highly reversible and can be considered benign. In general, NAFLD may be diagnosed by the combination of examinations such as conventional clinical examinations, for example, assay of liver-related enzymes in blood, abdominal echo, and abdominal CT. Unlike NAFLD, NASH is a reportedly difficult-to-detect disease based on such methods.
Other liver diseases, for example, viral hepatitis, can be detected or differentiated on the basis of the presence or absence of virus antigens or anti-virus antibodies in samples of patients according to established diagnostic criteria. In contrast to this, NASH is definitely examined only by the histopathological examination of livers involving evaluation based on liver biopsy, and only this method is clinically acceptable for the definite diagnosis of NASH at present.
The liver biopsy, however, usually requires hospitalization due to the excision of a portion of the liver itself for examination and is difficult to routinely carry out in terms of high economic burden and physical risk (e.g., bleeding) on patients. Meanwhile, simple steatosis, which must be differentially diagnosed from NASH, is a very common and benign disease and is often merely monitored over time. Nevertheless, under the circumstances, a positive suggestion has not yet been made about when physicians should biopsy the livers of patients who are diagnosed with NAFLD in routine clinical settings and further suspected of NASH. Patients may feel further largely burdened when diagnosed with simple steatosis as a result of liver biopsy, though this is due to medical needs and limitations. Thus, physicians often find difficulty in making a decision to perform liver biopsy.

Meanwhile, various methods other than liver biopsy for detecting or differentiating NASH have been attempted to reduce burden on patients.
Nevertheless, conventional diagnosis methods using a rise in the levels of liver-related enzymes such as serum aminotransferase are insufficient for distinguishing NASH from the other liver diseases such as simple steatosis. Specifically, the examined level of aminotransferase (e.g., alanine aminotransferase (ALT) or aspartate aminotransferase (AST)) that serves as an index for NASH is neither highly sensitive nor specific because it mostly overlaps with abnormal examined levels attributed to many diseases such as the other lifestyle-related disease, liver disease, blood disease, and malignant tumor. In addition, NASH is frequently found even when the levels of these enzymes fall within normal ranges.

Meanwhile, a method for diagnosing NASH has also been studied, which involves scoring conventional diagnostic markers (blood diagnostic markers ALT and AST as well as races, the presence or absence of diabetes mellitus, etc.) in combination (Non Patent Literature 3). In this method, however, approximately half of a group confirmed with "high" risk by the combined scoring of various markers actually had no NASH. On the other hand, a group confirmed with "intermediate" or "low" risk included a higher rate of non-NASH patients, but disadvantageously included nearly 30% of NASH patients even in this confirmation. Thus, this method presents problems in the accuracy of NASH diagnosis.
Alternatively, a method for differentiating NASH by ultrasound diagnosis has also been studied (Non Patent Literature 4). In this case, 9 out of 34 cases diagnosed with fatty liver had normal levels of conventional blood diagnostic markers (including ALT and AST as well as y glutamyl transpeptidase (γ-GTP) and alkaline phosphatase (ALP)), whereas 7 out of these 9 cases disclosed had NASH as a result of liver biopsy, indicating difficulty in NASH diagnosis using conventional diagnostic markers.
Under the circumstances, liver biopsy, which places large burden on patients, is only one definite diagnosis method for detecting or differentiating NASH, even though NASH in patients must be detected and treated at an early stage. Thus, there is a strong demand for a method for detecting or differentiating the disease using an easy-to-collect sample such as blood.

### Citation List

### Non Patent Literature

Non Patent Literature 1: Mayo Clin. Proc., 55: 434-438, 1980
Non Patent Literature 2: Gastroenterology, 121, 710-723 (2001)
Non Patent Literature 3: Hepatology, 47, 1916-1923 (2008)
Non Patent Literature 4: World J. Gastroenterol., 15 (15), 1863-1868 (2009)

### Summary of Invention

### Technical Problem

Under such situations, an object of the present invention to provide a marker for detection and/or differentiation of non-alcoholic steatohepatitis, which has previously been difficult to differentiate by an approach other than liver biopsy, a method for detecting and/or differentiating non-alcoholic steatohepatitis, and a kit for use in the method.

### Solution to Problem

The present inventor has conducted diligent studies to attain the object and consequently completed the present invention by finding that the level of alanine-glyoxylate aminotransferase (in the present specification, also abbreviated to AGXT) is significantly higher in non-alcoholic steatohepatitis patient-derived serum than that in healthy person-derived serum; thus alanine-glyoxylate aminotransferase can serve as a marker for detection and/or differentiation of non-alcoholic steatohepatitis.
Specifically, the present invention relates to:
[1] a marker for detection and/or differentiation of non-alcoholic steatohepatitis, consisting of alanine-glyoxylate aminotransferase;
[2] a method for detecting and/or differentiating non-alcoholic steatohepatitis, comprising assaying alanine-glyoxylate aminotransferase in a sample;
[3] a method for detecting and/or differentiating non-alcoholic steatohepatitis, comprising comparing the level of alanine-glyoxylate aminotransferase in a sample with that in a healthy person-derived sample and determining that the sample is derived from non-alcoholic steatohepatitis, when the level is higher;
[4] the method according to [2] or [3], wherein the alanine-glyoxylate aminotransferase is assayed by immunoassay using an antibody specifically binding to alanine-glyoxylate aminotransferase or by determination of the enzymatic activity of alanine-glyoxylate aminotransferase;
[5] the method according to any one of [2] to [4], wherein the sample is a blood-derived sample;
[6] a kit for detection and/or differentiation of non-alcoholic steatohepatitis, comprising an antibody specifically binding to alanine-glyoxylate aminotransferase or a reagent for assaying the enzymatic activity of alanine-glyoxylate aminotransferase;
[7] use of alanine-glyoxylate aminotransferase as a marker for detection and/or differentiation of non-alcoholic steatohepatitis; and
[8] alanine-glyoxylate aminotransferase for use as a marker for detection and/or differentiation of non-alcoholic steatohepatitis.

### Advantageous Effects of Invention

According to the present invention, NASH patients can be differentiated and distinguished from healthy persons and simple steatosis patients by the assay of alanine-glyoxylate aminotransferase in patient-derived samples. The samples may be ones conveniently collectable from patients, such as serum. This alanine-glyoxylate aminotransferase assay achieves accurate NASH diagnosis based on the convenient examination of the blood, though liver biopsy usually involving hospitalization has previously been considered essential for the definite diagnosis of NASH. Thus, the present invention can be applied to use in the field of diagnosis including diagnostic drugs. Unlike benign simple steatosis, NASH may be related to oncogenesis with a progressive course from hepatitis to liver cirrhosis, as pointed out. In this regard, there is a high need for the definite diagnosis of NASH. Non-alcoholic steatohepatitis can be detected and/or differentiated accurately using the marker for detection and/or differentiation of non-alcoholic steatohepatitis of the present invention. The marker for detection and/or differentiation of non-alcoholic steatohepatitis of the present invention can particularly discriminate between non-alcoholic steatohepatitis and simple steatosis, which have previously been difficult to differentiate by an approach other than liver biopsy, and by extension, discriminate between healthy persons and non-alcoholic steatohepatitis patients.

### Brief Description of Drawings

[Fig. 1] Fig. 1 shows results of Western blotting and quantification thereof conducted in Example 1 on sera from healthy male persons (Control) and male patients with non-alcoholic steatohepatitis (NASH) or with simple steatosis (FL).
[Fig. 2] Fig. 2 shows results of Western blotting and quantification thereof conducted in Example 1 on sera from healthy female persons (Control) and female patients with non-alcoholic steatohepatitis (NASH) or with simple steatosis (FL).

### Description of Embodiments

The marker for detection and/or differentiation of non-alcoholic steatohepatitis according to the present invention is alanine-glyoxylate aminotransferase. In the present invention, the detection of non-alcoholic steatohepatitis refers to the confirmation of the presence or absence of non-alcoholic steatohepatitis itself by investigation. The differentiation of non-alcoholic steatohepatitis refers to the discrimination between non-alcoholic steatohepatitis and the other disease, for example, simple steatosis by distinguishment. AGXT is represented by EC2.6.1.44 and is one of enzymes that catalyze the transfer of an amino group from alanine and glyoxylate to produce pyruvate and glycine. AGXT is known to exhibit localized expression particularly in the liver in vivo (Clin. Biochem., Vol. 18, October 1985, 311-316).
In the present invention, for example, the presence or absence of or the amount of the marker protein AGXT for NASH detection and/or differentiation can be detected or determined in a sample obtained from a patient suspected of NASH to detect the onset of NASH or differentiate NASH from the other liver disease such as simple steatosis.
The method for detecting and/or differentiating non-alcoholic steatohepatitis according to the present invention can comprise comparing the level of AGXT in a sample of a test subject with that in a healthy person-derived sample and determining that the test subject has non-alcoholic steatohepatitis, when the level is higher. More specifically, the method of the present invention is characterized by comprising, for example, the steps of: (i) determining the level of AGXT in an appropriate sample derived from an individual to be diagnosed; and (ii) comparing the determined level with the corresponding level (normal level or normal concentration range) in an evidently healthy individual (normal individual). The individual is suspected of NASH when the determined level of AGXT is high out of the normal level.
In the present invention, the alanine-glyoxylate aminotransferase can be used as a marker for detection and/or differentiation of non-alcoholic steatohepatitis, as described above.

Examples of the sample that can be used in the present invention include blood-derived samples, for example, serum, plasma, and blood, and urine collected from patients suspected of the disease NASH. The sample is preferably a blood-derived sample. The sample is particularly preferably a sample of a patient who has been proved free from viral hepatitis, but has not been found to be a general simple steatosis patient or an NASH patient.

In the present invention, the detection of the presence or absence of AGXT or the determination of the amount thereof can adopt, as a rule, any currently known method with sensitivity, accuracy, specificity, etc., to be satisfied. Examples of the method can include the assay of the AGXT protein by immunoassay and the determination of the enzymatic activity of AGXT by an enzymatic method. Immunoassay is preferable because of its high assay sensitivity, possible specific assay, etc.
The immunoassay is characterized by contacting a sample possibly containing AGXT with an antibody specifically binding to AGXT (anti-AGXT antibody) under conditions capable of forming an immune complex consisting of AGXT and the anti-AGXT antibody. Then, the formed complex can be assayed by a method known in the art to obtain the quantitatively or qualitatively determined level of AGXT in the sample, i.e., the determined level of AGXT in the sample.
In these assay systems, the immune complex can be assayed directly. Alternatively, this complex can be assayed on the basis of a label with an analytically detectable substance (labeling material). The labeling material can be incorporated specifically in the immune complex.

Examples of the immunoassay can include previously known protein assay methods using polyclonal or monoclonal antibodies against AGXT, which is the marker protein for detection and/or differentiation of non-alcoholic steatohepatitis of the present invention. Specific examples of such immunoassay can include enzyme immunoassay (EIA), turbidimetric immunoassay (TIA), latex immunoagglutination assay (LATEX), electrochemiluminescent immunoassay, fluorescent immunoassay, and Western blotting. Also, immunochromatography or a method using a test paper is effective. Any of these methods are well known per se to those skilled in the art, and these well known methods can be adopted directly in the present invention.
A commercially available antibody (e.g., ABGENT rabbit anti-human AGXT antibody) can be used in the immunoassay. Further examples of the antibody that can be used in the immunoassay include polyclonal and monoclonal antibodies prepared by methods already routinely used. The AGXT antigen for preparation of the antibody may be obtained by purification from human blood or may be synthesized by a genetic engineering technique based on the amino acid sequence of AGXT known in the art or a gene sequence encoding it (Biochem. Biophys. Res. Commun., 176 (3): 1093-1099, July 1991; and Genomics, 10 (1): 34-42, July 1991). Alternatively, the antigen may be obtained by chemical synthesis using a peptide synthesis technique known in the art.

Hereinafter, enzyme immunoassay will be described as an example. First, monoclonal antibodies against AGXT are bonded directly or indirectly to a solid phase known per se in the art, such as polystyrene, polypropylene, polycarbonate, polyethylene, nylon, or polymethacrylate, through the use of physical bond, chemical bond, or affinity. The amount of the sensitizing antibodies is usually in the range of 1 ng to 100 mg/ml. A sample is added to the monoclonal antibodies bonded to the solid phase through physical bond, chemical bond, affinity, or the like, and reacted therewith. After the reaction for a predetermined time, the solid phase is washed and further secondarily reacted by the addition of corresponding labeled secondary antibodies (e.g., labeled secondary anti-AGXT antibodies). The solid phase is washed again and reacted by the addition of a chromogenic substrate DAB or the like. In the case of using HRP as a labeling material, known DAB, TMB, or the like can be used as a substrate, though the labeling material is not limited to this. Examples thereof include enzymes as well as any distinguishable substance including labeling metals such as gold colloid and europium, various chemical or biological fluorescent materials such as FITC, rhodamine, Texas Red, Alexa, and GFP, radioactive materials such as ³²P and ⁵¹Cr.

The assay of AGXT by Western blotting separates AGXT from various substances in samples through the excellent resolution capability of gel electrophoresis combined with the high specificity of antigen-antibody reaction and detects the AGXT or determines the amount thereof. Specifically, samples are first subjected to SDS-PAGE, isoelectric focusing, or two-dimensional electrophoresis. Subsequently, AGXT is electrically transferred and immobilized to a membrane from the gel to prepare a blot (membrane). The membrane used is preferably made of highly hydrophobic nitrocellulose or polyvinylidene fluoride (PVDF) more excellent in hydrophobicity, to which the protein is capable of easily binding. Then, the blot is reacted with anti-AGXT antibodies for detection. The detection method may involve a system using an enzyme, fluorescence, etc. In the system using an enzyme, secondary antibodies labeled with ALP, HRP, or the like are reacted with the primary antibodies and detected by color development or chemiluminescence based on enzymatic activity. Alternatively, the detection based on chemiluminescence can detect AGXT or determine the level thereof by exposure to x-ray films or by the detection of the chemiluminescence using a scanner. In the case of using the fluorescent immunoassay, AGXT may be quantified using secondary antibodies labeled with Cy3 or Cy5.

Examples of the method for determining the enzymatic activity of AGXT by the enzymatic method include a method which involves reacting AGXT with its substrates and assaying the formed product to thereby determine the AGXT activity. Specific examples thereof include a method which involves reacting the enzyme with its substrates alanine and glyoxylate as essential components, optionally in the presence of pyridoxal phosphate, and assaying the formed pyruvate. Examples of the method for assaying the formed pyruvate include: a reductive method which involves allowing lactate dehydrogenase and NADH to act on the pyruvate and determining the amount of NADH decreased to assay the pyruvate; and an oxidative method which involves allowing phosphate and pyruvate oxidase to act on the pyruvate and assaying the formed hydrogen peroxide to assay the pyruvate.

In the case of the reductive method, lactate dehydrogenase and NADH are allowed to act on the pyruvate. The reaction solution is irradiated with ultraviolet rays having a wavelength of 340 nm that is absorbed by NADH. The amount of NADH decreased can be determined on the basis of the rate of decrease in absorbance to assay the pyruvate.

In the case of the oxidative method, the amount of hydrogen peroxide is preferably determined by the assay of a dye formed using peroxidase and a substrate that develops color via oxidation. In this context, examples of the substrate that develops color via oxidation include a substrate composed of a single compound that develops color via oxidation, and a substrate composed of a Trinder reagent in combination with 4-aminoantipyrine or the like. Examples of the single compound that develops color via oxidation can include leuco dyes such as N-carboxymethylaminocarbonyl)-4,4'-bis(dimethylamino)-diphenylamine sodium salt (DA-64) and 10-(carboxymethylaminocarbonyl)-3,7-bis(dimethylamino)-phenothiazine sodium salt (DA-67), N,N,N`N',N",N"-hexa(3-sulfopropyl)-4,4',4",-triamino-triphenylmethane hexasodium salt (TPM-PS), and ortho-phenylenediamine (OPD). Examples of the Trinder reagent include phenol derivatives and aniline derivatives. Specific examples of the Trinder reagent include 2,4-dichlorophenol, N-ethyl-N-(2-hydroxy-3-sulfopropyl)-3,5-dimethoxyaniline (DAOS), N-ethyl-N-sulfopropyl-3,5-dimethylaniline (MAPS), N-ethyl-N-(2-hydroxy-3-sulfopropyl)-3,5-dimethylaniline (MAOS), N-ethyl-N-(2-hydroxy-3-sulfopropyl)-m-toluidine (TOOS), N-ethyl-N-sulfopropyl-m-anisidine (ADPS), N-ethyl-N-sulfopropylaniline (ALPS), N-ethyl-N-sulfopropyl-3,5-dimethoxyaniline (DAPS), N-sulfopropyl-3,5-dimethoxyaniline (HDAPS), N-ethyl-N-sulfopropyl-m-toluidine (TOPS), N-ethyl-N-(2-hydroxy-3-sulfopropyl)-m-anisidine (ADOS), N-ethyl-N-(2-hydroxy-3-sulfopropyl)aniline (ALOS), N-(2-hydroxy-3-sulfopropyl)-3,5-dimethoxyaniline (HDAOS), and N-sulfopropyl-aniline (HALPS).
Also, an aminoantipyrine derivative, vanillin diamine sulfonic acid, methyl benzothiazolinone hydrazone (MBTH), sulfonated methyl benzothiazolinone hydrazone (SMBTH), or the like can be used as a compound corresponding to 4-aminoantipyrine described above.

The method for detecting and/or differentiating non-alcoholic steatohepatitis according to the present invention can be carried out using, for example, a kit for detection and/or differentiation of non-alcoholic steatohepatitis, comprising at least an antibody specifically binding to AGXT (anti-AGXT antibody) as a reagent component or a reagent for assaying the enzymatic activity of AGXT.
The anti-AGXT antibody can be used as a reagent component in the kit, for example, in a form bonded to a water-insoluble carrier such as a microplate. Examples of other reagent components in the kit include a secondary antibody such as an anti-human immunoglobulin antibody. This secondary antibody used is labeled with a labeling material such as an enzyme, a radioisotope, a fluorescent material, or a chemiluminescent material according to an adopted assay method such as enzyme immunoassay, radioimmunoassay, fluorescent immunoassay, or chemiluminescent immunoassay. The kit may be supplemented appropriately with other reagent components such as a surfactant and a buffer.

Examples of the reagent for assaying the enzymatic activity of AGXT include AGXT substrates and a reagent for assaying a product formed through reaction with the substrates. Such a reagent that can be used comprises substrates alanine and glyoxylate, and components for assay of pyruvate to be formed as essential components, and optionally, pyridoxal phosphate. The components for pyruvate assay can include lactate dehydrogenase and NADH for the reductive method and include phosphate, pyruvate oxidase, and components for hydrogen peroxide assay for the oxidative method. The components for hydrogen peroxide assay can include peroxidase and a substrate that develops color via oxidation. Examples of the substrate that develops color via oxidation can include the substrate composed of a single compound that develops color via oxidation, and the combination of a Trinder reagent with 4-aminoantipyrine or the like, as exemplified above.

The method described above is capable of detecting and/or differentiating NASH by detecting the presence or absence of AGXT or determining the amount thereof in a sample obtained from a patient suspected of NASH.
The method of the present invention can differentiate NASH patients from healthy individuals and can also perform the differentiation of NASH patients from FL patients, which has previously been difficult to achieve by an approach other than liver biopsy.

### Examples

Hereinafter, the present invention will be described more specifically with reference to Examples. However, the present invention is not intended to be limited to these Examples by any means.

### Example 1

### Determination of alanine-glyoxylate aminotransferase (AGXT) levels in sera of non-alcoholic steatohepatitis (NASH) patients simple steatosis (FL) patients, and healthy persons

The respective sera of 18 healthy persons (10 males and 8 females), 9 FL patients (4 males and 5 females), and 17 NASH patients (8 males and 9 females) were examined for their serum AGXT levels by a method described below. Comparison among the healthy persons, the NASH patients, and the FL patients were conducted without statistically large bias in sex, age, and body mass index (BMI).
The healthy person sera were collected on empty stomachs from volunteers with written consent from among persons who received health checks. All cases of the NASH patients were definitely diagnosed by liver biopsy in hospitalization for routine tests. Their sera were obtained on empty stomachs under consent during the procedures. The FL patients were hepatically found to have fatty liver by liver biopsy or liver echo examination. Their sera were obtained on empty stomachs under consent during the procedures.

### 1. Method

For serum AGXT assay, 0.2 µL of untreated serum was suspended by the addition of 50 µL of 2 × SDS sample buffer (0.125 M tris-Cl, 2.0% sodium dodecyl sulfate (SDS), 10% glycerol, 0.05% bromophenol blue, pH 6.8) and pure water. A 10 µL aliquot of the suspension was used to perform polyacrylamide gel electrophoresis (SDS-PAGE). The gel used in SDS-PAGE was a polyacrylamide gel (DRC Co., Ltd.) of 8 cm long, 14 cm wide, and 1 mm thick having 10 to 20% acrylamide concentration gradients. Subsequently, Western blotting was conducted according to a method known in the art. Specifically, proteins separated by SDS-PAGE were transferred to a polyvinylidene fluoride (PVDF) membrane. The PVDF membrane after transfer was cut into a predetermined size and subjected to Ponceau S staining to confirm the absence of variations in transfer. Subsequently, the PVDF membrane was blocked by overnight dipping at 4°C in 0.05% skimmed milk-phosphate with Tween 20 (PBS-T) and subsequently washed by dipping in PBS-T at room temperature for 10 minutes. This procedure was repeated three times. The PVDF membrane thus washed was incubated at room temperature for 1 hour in a solution of rabbit anti-human AGXT antibodies (ABGENT) dissolved in PBS, and subsequently washed by dipping in PBS-T at room temperature for 5 minutes. This procedure was repeated three times. The PVDF membrane thus washed was incubated at room temperature for 1 hour in PBS containing HRP-labeled anti-rabbit IgG antibodies (Dako), and subsequently washed by dipping in PBS-Tween 20 (0.1%) at room temperature for 5 minutes. This procedure was repeated three times. Luminescence signals from the PVDF membrane thus washed were detected using FEMTOGLOW Western blotting luminescence reagent (Michigan Diagnostics LLC) according to the accompanying protocol. The signal detector and image analyzer used were LumiVision PRO 400EX (Aisin Seiki Co., Ltd.). The luminescence signals were photographed and digitized.

### 2. Results

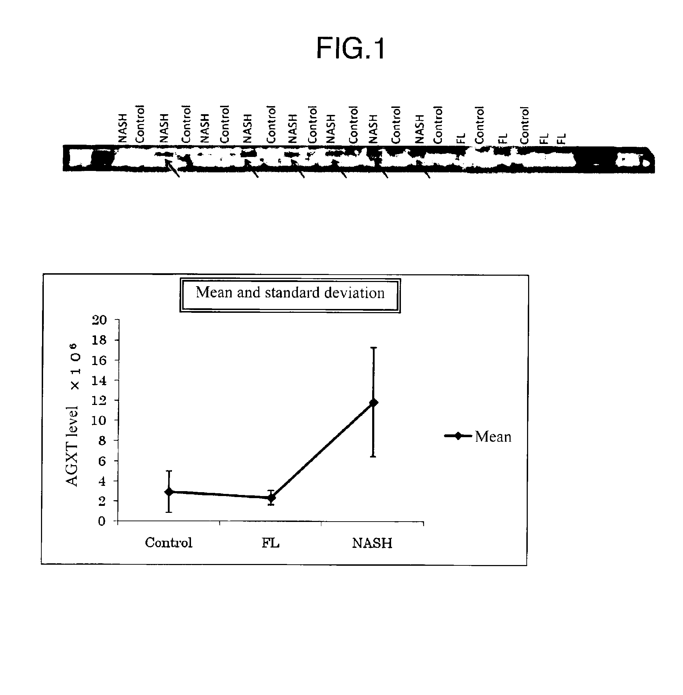

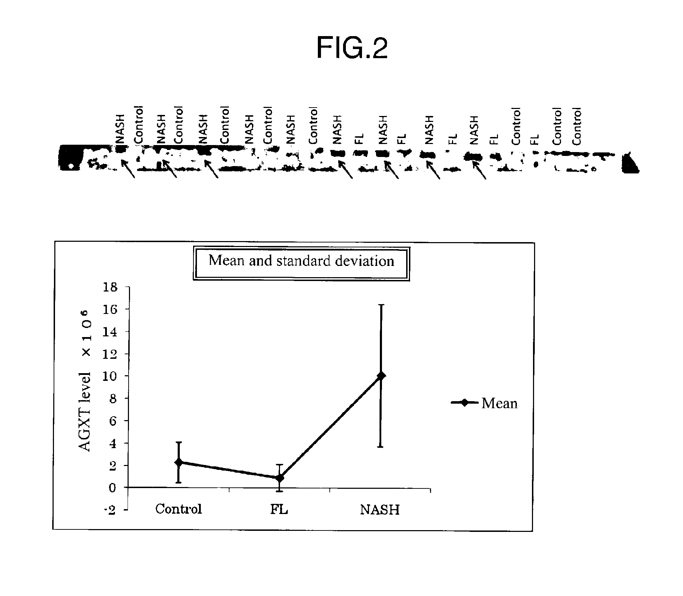

The results of image analysis of the signals obtained by Western blotting are shown in Fig. 1 for males and in Fig. 2 for females. Also, the signal intensity of each band in the image analysis results was digitized for both males and females to determine AGXT levels. The results are as shown in Table 1.

**[Table 1]**

| Comparison of AGXT levels by Western blotting | | | |
|---|---|---|---|
| AGXT level (× 10⁶) | Healthy control | FL patient | NASH patient |
| 7.5 or more | 0 persons | 0 persons | 14 persons |
| 7.5 or less | 18 persons | 9 persons | 3 persons |

These results demonstrated that NASH patients can be differentiated from healthy individuals and FL patients with high accuracy by AGXT assay.

### Industrial Applicability

The levels of existing liver function markers such as GOT (AST) or GPT (ALT) are easily elevated due to simple steatosis, non-NASH liver diseases, or diseases other than these liver diseases, whereas AGXT has specificity for NASH and a much lower risk of misdiagnosis and thus make a great contribution to the field of diagnosis. Accordingly, AGXT can be assayed as a marker for detection and/or differentiation of non-alcoholic steatohepatitis to thereby highly accurately detect or differentiate NASH patients from healthy individuals and simple steatosis patients.

## Claims

1. A marker for detection and/or differentiation of non-alcoholic steatohepatitis, consisting of alanine-glyoxylate aminotransferase.

2. A method for detecting and/or differentiating non-alcoholic steatohepatitis, comprising assaying alanine-glyoxylate aminotransferase in a sample.

3. A method for detecting and/or differentiating non-alcoholic steatohepatitis, comprising comparing the level of alanine-glyoxylate aminotransferase in a sample with that in a healthy person-derived sample and determining that the sample is derived from non-alcoholic steatohepatitis, when the level is higher.

4. The method according to claim 2 or 3, wherein the alanine-glyoxylate aminotransferase is assayed by immunoassay using an antibody specifically binding to alanine-glyoxylate aminotransferase or by determination of the enzymatic activity of alanine-glyoxylate aminotransferase.

5. The method according to any one of claims 2 to 4, wherein the sample is a blood-derived sample.

6. A kit for detection and/or differentiation of non-alcoholic steatohepatitis, comprising an antibody specifically binding to alanine-glyoxylate aminotransferase or a reagent for assaying the enzymatic activity of alanine-glyoxylate aminotransferase.

7. Use of alanine-glyoxylate aminotransferase as a marker for detection and/or differentiation of non-alcoholic steatohepatitis.

8. Alanine-glyoxylate aminotransferase for use as a marker for detection and/or differentiation of non-alcoholic steatohepatitis.
